Europäisches Patentamt

European Patent Office

Office européen des brevets

(19)

(11) Numéro de publication : **0 196 434 B1**

# FASCICULE DE BREVET EUROPÉEN

(12)

(45) Date de publication du fascicule du brevet :
28.06.89

(51) Int. Cl.⁴ : **A 61 K   9/14**, A 61 K 47/00,
   **A 23 G   1/00**

(21) Numéro de dépôt : **86101951.1**

(22) Date de dépôt : **15.02.86**

(54) Procédé pour la préparation d'une composition à base d'un principe actif de faible hydrosolubilité finement divisé.

(30) Priorité : **20.03.85 CH 1235/85**

(43) Date de publication de la demande :
   **08.10.86 Bulletin 86/41**

(45) Mention de la délivrance du brevet :
   **28.06.89 Bulletin 89/26**

(84) Etats contractants désignés :
   **AT BE DE FR IT LU NL SE**

(56) Documents cités :
   **BE–A–   442 390**
   **DE–A–   868 491**
   **GB–A– 1 235 540**
   **US–A– 3 243 347**
   **THE MERCK INDEX, édition 2, 1983, Merck & Co., Inc.,**
   **Rahway, N.J., US.**

(73) Titulaire : **SOCIETE DES PRODUITS NESTLE S.A.**
   **Case postale 353**
   **CH-1800 Vevey (CH)**

(72) Inventeur : **Hirsbrunner, Pierre**
   **El Cantero Chemin des Romains**
   **CH-1801 Les Monts-de-Corsier (CH)**

EP 0 196 434 B1

## Description

L'invention concerne un procédé pour la préparation d'une composition à base d'un principe actif de faible hydrosolubilité et finement divisé, apte à être dispersée dans l'eau sans former de dépôt.

Le problème du dépôt ou de la sédimentation d'un principe actif sensiblement hydroinsoluble lors de la reconstitution dans l'eau est connu depuis longtemps. Il se pose avec une extrême acuité, surtout lorsqu'il s'agit de médicaments. En effet, le patient souhaitant ingérer un médicament doit suivre la posologie de son médecin traitant. Si lors de la reconstitution dans l'eau, une fraction du principe actif reste au fond du verre et si l'opération se répète plusieurs fois par jour, il y a un grand risque d'inopérance du traitement envisagé. Diverses solutions ont été proposées pour éliminer cet inconvénient. On ajoute par exemple à de telles compositions pharmaceutiques ou alimentaires des émulsifiants ou des agents de dispersion qui favorisent ainsi la mise en suspension du principe actif. L'addition de ces additifs n'est cependant pas permise par la législation de tous les pays, de sorte qu'il faut prévoir diverses préparations suivant les pays dans lesquels on souhaite vendre. D'autre part, cette addition augmente le prix de la composition envisagée.

Le brevet BE 442390 concerne un procédé de fabrication de pièces moulées à partir de lait en poudre, de cacao en poudre ou de café en poudre et de sucre, dans lequel on soumet ce mélange à une pression inférieure à 150 kg/cm². Ce type de traitement améliore la tenue des comprimés, mais ne concerne pas le problème de la stabilité d'une suspension lors de la reconstitution dans l'eau.

Le procédé, objet de la présente invention, permet d'éliminer l'inconvénient d'ajouter une substance supplémentaire dans une composition prévue pour être dispersée dans l'eau, tout en améliorant ladite dispersabilité dans l'eau.

L'invention concerne un procédé pour la préparation d'une composition à base d'un principe actif de faible hydrosolubilité et finement divisé, apte à être dispersée dans l'eau, sans former de dépôt, dans lequel on mélange le principe actif à raison de 0,4 à 10 % en poids avec des solides lactiques non-gras, de préférence de la poudre de lait écrémé et dans lequel on met en mouvement les particules dudit mélange à une température supérieure à 40 °C.

Il est important selon l'invention qu'il y ait concomittance du mouvement des particules du mélange et du traitement thermique.

Le principe actif hydroinsoluble traité n'est pas critique. Il peut s'agir de toute substance en poudre sensiblement non soluble dans l'eau et utilisable sous forme de dispersion dans l'eau. Cela peut être un principe pharmacoactif ou un composé utilisable à des fins alimentaires. Comme principe pharmacoactif, on envisage notamment des anti-inflammatoires systémiques, tels ceux objets de la demande de brevet DE-OS 2 537 070 ou leurs homologues, en particulier le produit dénommé Tenoxicam, ou ceux objet du brevet US 3 591 584 ou leurs homologues, en particulier le Piroxicam.

Comme principe actif alimentaire, on utilise de préférence selon l'invention le cacao ou la caroube.

Par finement divisé on entend des particules ayant des dimensions comprises entre environ 0,2 et 40 micromètres.

Le problème de la sédimentation de produits faiblement hydroinsolubles se trouve encore accru si on utilise des produits ayant un poids spécifique élevé. C'est le cas notamment du Tenoxicam précité. Le produit actif se dépose alors au fond du récipient plus rapidement, augmentant encore les risques de fausse posologie. En outre, avec un principe pharmacoactif, il est également connu que ceux-ci peuvent occasionner chez les patients des troubles d'ordre gastrique. La poudre de lait a déjà été proposée pour atténuer cette incompatibilité (DE 2519528). On utilise de préférence de la poudre de lait écrémé instantanée ayant une teneur en eau comprise entre 2 et 4 %.

La température à laquelle on effectue la mise en mouvement des particules du mélange est critique dans le procédé selon l'invention. En effet, si la température est trop basse, il n'y a pas de phénomène de « piégeage » ou d' »adsorption » du principe actif par la poudre de lait. Par contre, si la température est trop élevée, on assiste à un « brûlage » de la poudre de lait. De préférence, la température de traitement est comprise entre 60 et 90 °C.

Lorsque le cacao est le principe actif, on mélange de préférence directement toute la quantité de poudre de lait écrémé instantanée avec le cacao ; on mélange alors 5 à 10 % en poids de cacao avec la poudre de lait.

Lorsque le principe actif est une substance à activité pharmacologique, on la mélange à raison de 0,4 à 10 % en poids avec la poudre de lait et on rajoute ensuite de la poudre de lait écrémé de manière à obtenir une concentration en principe actif comprise entre 0,1 et 1 % en poids.

Selon cette forme de mise en œuvre du procédé selon l'invention, on opère en deux étapes : dans la première, on mélange le principe actif avec une partie de la poudre de lait et on soumet le mélange au traitement thermique et dans la seconde on rajoute le complément de poudre de lait de manière à obtenir la concentration souhaitée en principe actif.

Il est bien entendu que la forme de mise en œuvre pour le principe actif alimentaire peut être envisagée pour le principe pharmacoactif et inversement.

Outre la température de traitement, il est important selon l'invention de mettre en mouvement les particules du mélange ; on soumet ces particules soit à un traitement brutal et rapide soit à un traitement long et doux.

Selon le premier mode de mise en œuvre, on met en mouvement les particules du mélange à l'aide d'un moyen mécanique, de préférence un moulin électrique à pales, par exemple à l'aide d'une moulinette électrique. Ce traitement dure entre 15 et 120 secondes ; compte tenu de l'échauffement du moteur, le mélange subit de manière concomitante un traitement thermique à une température comprise entre 60 et 90 °C.

Selon la seconde forme de mise en œuvre, le mélange est mis en mouvement par un moyen d'agitation, par exemple avec un agitateur magnétique. Dans ce cas, le mélange de particules est mis dans un bain-marie à une température comprise entre 50 et 80 °C et le traitement dure entre 15 et 120 minutes.

Lorsqu'on opère conformément au procédé de l'invention, on obtient une poudre dans laquelle les particules de lait ont au moins partiellement encapsulé la substance active, permettant ainsi lors de la reconstitution dans l'eau d'obtenir une suspension suffisamment stable pendant une durée bien supérieure à celle prévue pour l'ingestion de ladite suspension. On peut également ajouter que la poudre obtenue se prête bien au stockage pendant une durée équivalente à celle du lait en poudre seul.

L'invention concerne en outre le produit obtenu selon le procédé décrit ci-dessus. Ce produit se présente sous la forme de poudre ou de tablettes. Dans le cas d'un principe actif alimentaire, on préfère la forme de poudre tandis que dans le cas du principe pharmacoactif, le produit se présente plutôt sous forme de tablettes.

Que le produit soit sous la forme de poudre ou de tablettes, il peut également contenir tout additif connu dans la technique, par exemple des aromatisants, des édulcorants tels sucres, aspartame et autres ou tous les excipients connus et utilisables dans des compositions pharmaceutiques, tels ceux utilisés par exemple dans les compositions anti-inflammatoires. La quantité de ces additifs utilisés est celle connue dans la technique.

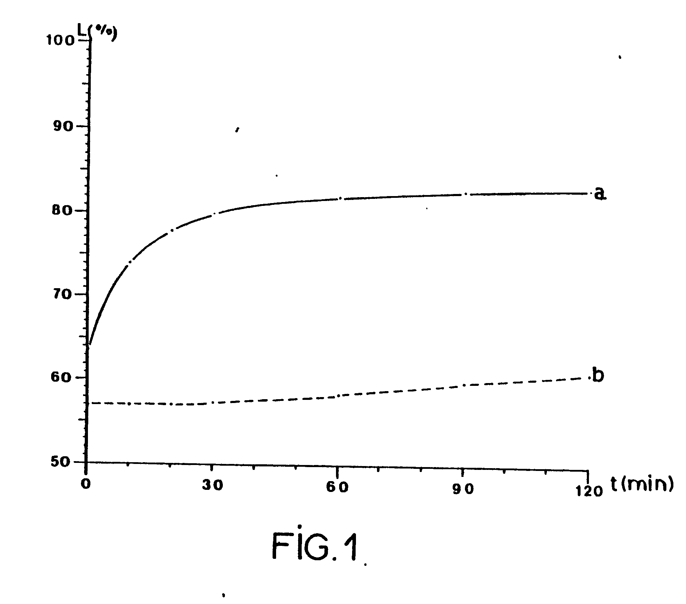

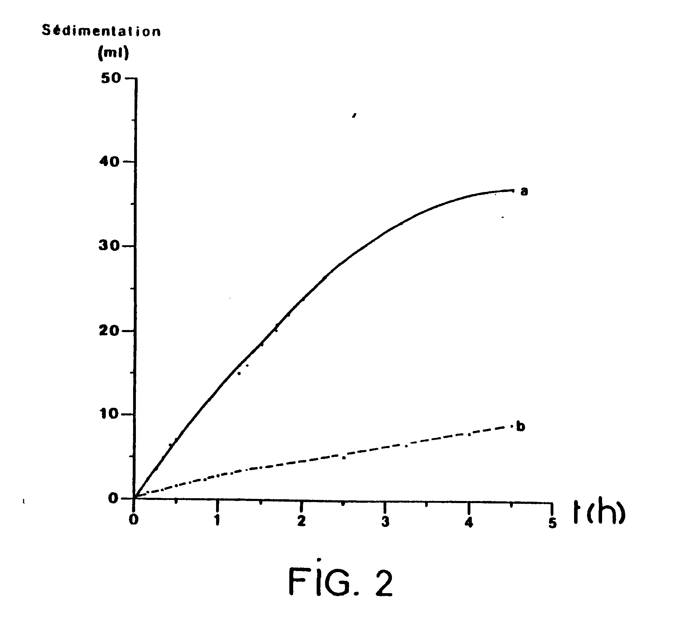

La suite de la description est faite en référence aux exemples et aux figures sur lesquelles Fig 1 et 2 représentent la sédimentation en fonction du temps, analysée avec un spectrocolorimètre et selon le test d'Imhof.

Exemple 1

27 g de poudre de lait écrémé instantanée sont introduits avec 3 g de poudre de cacao contenant 17 % de lipides dans un moulin électrique à pales à rotation rapide. Après 90 secondes de fonctionnement la température de la poudre est de 82 °C; la structure primaire du lait en poudre, bien que profondément modifiée, permet une dissolution rapide dans l'eau et confère au cacao le pouvoir de rester en suspension pendant un délai remarquablement plus long que dans la référence.

Pour mettre en évidence ce délai, on effectue un test de sédimentation au spectrocolorimètre. Le principe de ce test est basé sur la mesure de l'intensité lumineuse d'un faisceau de lumière réfléchi après avoir traversé l'échantillon à étudier.

Le facteur L désigne cette intensité lumineuse réfléchie. On étalonne le spectrocolorimètre utilisé, à savoir un appareil PYE-UNICAM Mod. SP-8/100, avec du lait entier et on affecte à celui-ci la valeur L = 100 %. La valeur L = 0 serait l'absence totale de réflexion, à savoir avec un corps noir. Le faisceau incident a une longueur d'onde de 546 nm.

La Fig 1 montre la variation du facteur L en fonction du temps pour la référence (courbe a) et selon l'exemple 1 (courbe b). La référence comporte 10 % de cacao avec de la poudre de lait écrémé instantanée mélangés à froid. Le mélange selon le présent exemple contient la même quantité de cacao, mais traité conformément à l'invention. Une valeur L petite signifie que les particules sont en suspension, par contre L élevée indique une sédimentation.

Pour la référence, on voit bien une augmentation rapide de L, tandis que pour l'échantillon selon l'exemple 1 le facteur L ne varie que très peu. Dans le premier cas, il y a donc sédimentation rapide du cacao tandis que pour l'échantillon selon l'invention il y a très peu de sédimentation au bout de deux heures.

Le test d'Imhof permet également de mettre en évidence la sédimentation d'un produit en suspension. Dans ce cas, on mélange 100 g de matière sèche avec 900 ml d'eau et on étudie la sédimentation S en ml sur un tube à essai de forme effilée. La figure 2 indique les résultats de cet essai pour la référence (courbe a) et pour l'échantillon conforme à l'invention (courbe b). La différence est extrêmement nette : la référence indique une sédimentation de 40 % au bout de 5 heures alors qu'elle n'est que de 10 % pour l'échantillon selon l'invention.

Exemple 2

90 g de poudre de lait écrémé instantanée et 10 g de poudre de cacao sont simultanément introduits dans un récipient de verre muni d'un agitateur magnétique, lequel est soumis à une vitesse de rotation de 180 t/min. L'ensemble est thermostatisé dans un bain-marie de 60 °C agité dans les conditions décrites pendant 20 minutes.

Le produit obtenu ainsi a pratiquement conservé sa structure, mais au cours de la reconstitution dans l'eau, le cacao insoluble est maintenu en suspension comme dans l'exemple 1.

Exemple 3

3 g de Tenoxicam sont traités avec 27 g de poudre de lait écrémé instantanée dans les conditions décrites dans l'exemple 1. Après 90 secondes de traitement, on mesure une température de 85 °C et on obtient une poudre très homogène et fine. Par mélange à sec de 4 g de cette poudre avec 96 g de poudre de lait écrémé instantanée, on obtient une préparation granulaire de couleur jaune/crème, contenant 20 mg de Tenoxicam par dose de 5 g (dose thérapeutique) avec la particularité d'une stabilité parfaite de la suspension, lors de la dispersion de la préparation dans l'eau. L'amertume induite par la forte dispersion du principe actif amer peut être atténuée par l'usage d'aspartame (25 mg/dose), d'agent aromatisant (vanilline 10 mg/dose) et d'environ 1 % d'agent gélifiant (carboxy-méthyl-cellulose de Hercules Powders).

Dans la référence non traitée, le Tenoxicam est sédimenté à 45 % après 15 min, à 95 % après 30 min de la mise en suspension dans l'eau.

Exemple 4

8 g de Piroxicam (dimension de particules comprises entre 0,2 et 4 μm ont été introduits dans un réacteur en verre, avec 92 g de poudre de lait écrémé instantanée. Après 20 minutes de traitement sous agitation magnétique douce (comme dans l'exemple 2) dans un bain-marie de 70 °C, on obtient un mélange granulaire fortement coloré, lequel dilué par mélange à sec ordinaire dans 600 g de poudre de lait instantané écrémé et 300 g de caséine condensée au formol (produit de marque PLASVITA de Dynamit-Nobel) est tablettisé par les méthodes habituelles, la tablette de 2,5 g contenant 20 mg de principe actif, finement dispersé.

Sa 'mise en contact avec l'eau permet l'obtention d'une dispersion rapide et le principe actif coloré ne sédimente pas, même après un temps prolongé. On utilise un agent édulcorant et un agent aromatisant comme dans l'exemple 3.

Dans tous les exemples précités, la teneur en eau de la poudre de lait écrémé instantanée était de 3,5 %.

**Revendications**

1. Procédé pour la préparation d'une composition à base d'un principe actif de faible hydrosolubilité et finement divisé, apte à être dispersée dans l'eau sans former de dépôt, caractérisé en ce qu'on mélange le principe actif à raison de 0,4 à 10 % en poids avec de la poudre de lait écrémé et en ce qu'on met en mouvement les particules dudit mélange à une température supérieure à 40 °C, ledit principe actif ayant une dimension de particules comprise entre 0,2 et 40 μm.

2. Procédé selon la revendication 1, caractérisé en ce que le principe actif est choisi dans le groupe formé par le cacao, la caroube et un principe pharmacoactif tel qu'un anti-inflammatoire systémique.

3. Procédé selon la revendication 2, caractérisé en ce que l'anti-inflammatoire systémique est choisi dans le groupe formé par le Tenoxicam, le Piroxicam et leurs homologues.

4. Procédé selon l'une des revendications 1 ou 2, caractérisé en ce qu'on met en mouvement les particules du mélange à une température comprise entre 60 et 90 °C.

5. Procédé selon l'une des revendications 1 ou 2, caractérisé en ce qu'on utilise de la poudre de lait écrémé instantanée ayant une teneur en eau comprise entre 2 et 4 %.

6. Procédé selon la revendication 2, caractérisé en ce qu'on mélange 5 à 10 % de cacao avec la poudre de lait écrémé.

7. Procédé selon l'une des revendications 2 ou 3, caractérisé en ce qu'on mélange 0,4 à 10 % en poids de principe pharmacoactif avec de la poudre de lait écrémé et en ce qu'on rajoute ensuite de la poudre de lait écrémé de manière à obtenir une concentration en principe actif comprise entre 0,1 et 1 % en poids.

8. Procédé selon l'une des revendications 1 ou 2, caractérisé en ce qu'on met en mouvement les particules du mélange à l'aide d'un moulin électrique à pales pendant une durée comprise entre 15 et 120 secondes.

9. Procédé selon l'une des revendications 1 ou 2, caractérisé en ce qu'on met en mouvement les particules du mélange à l'aide d'un moyen d'agitation modérée pendant une durée comprise entre 15 et 120 minutes.

**Claims**

1. A process for the preparation of a composition based on a finely divided active principle sparingly soluble in water and intended to be dispersed in water without forming a deposit, characterized in that the active principle is mixed in a quantity of from 0.4 to 10 % by weight with skimmed milk powder, and in that the particles of said mixture are subjected to movement at a temperature above 40 °C, said active principle having a particle size ranging from 0,2 to 40 μm.

2. A process as claimed in Claim 1, characterized in that the active principle is selected from the group comprising cocoa and carob beans and a pharmacologically active principle, such as a systemic anti-inflammatory.

3. A process as claimed in Claim 2, characterized in that the systemic anti-inflammatory is selected from the group comprising Tenoxicam, Piroxicam and their homologs.

4. A process as claimed in Claim 1 or 2, characterized in that the particles of the mixture are subjected to movement at a temperature of from 60 to 90 °C.

5. A process as claimed in Claim 1 or 2, characterized in that instant skimmed milk powder having a water content of from 2 to 4 % is used.

6. A process as claimed in Claim 2, characterized in that from 5 to 10 % of cocoa is mixed with the skimmed milk powder.

7. A process as claimed in Claim 2 or 3, characterized in that from 0.4 to 10 % by weight of pharmacologically active principle is mixed with skimmed milk powder and in that skimmed milk powder is then added so as to obtain a concentration of active principle of from 0.1 to 1 % by weight.

8. A process as claimed in Claim 1 or 2, characterized in that the particles are subjected to movement by means of an electrical blade mill for between 15 and 120 seconds.

9. A process as claimed in Claim 1 or 2, characterized in that the particles of the mixture are subjected to movement by moderate agitation for between 15 and 120 minutes.

## Patentansprüche

1. Verfahren zur Herstellung einer Zusammensetzung auf der Basis eines feinverteilten Wirkstoffes von geringer Wasserlöslichkeit, die im Wasser ohne Ausbildung eines Niederschlages dispergiert werden kann, dadurch gekennzeichnet, daß man den Wirkstoff in einem Verhältnis von 0,4 bis 10 Gewichts-% mit Magermilchpulver vermischt und daß man die Teilchen dieses Gemisches bei einer Temperatur von über 40 °C in Bewegung versetzt, wobei der genannte Wirkstoff eine Teilchengröße zwischen 0,2 und 40 μm aufweist.

2. Verfahren nach Anspruch 1, dadurch gekennzeichnet, daß der Wirkstoff aus einer Gruppe ausgewählt wird, die aus Kakao, Johannisbrot und einem pharmazeutischen Wirkstoff wie einem entzündungshemmenden systemischen Wirkstoff besteht.

3. Verfahren nach Anspruch 2, dadurch gekennzeichnet, daß der entzündungshemmende systemische Wirkstoff aus der aus Tenoxicam, Piroxicam und deren Homologen gebildeten Gruppe ausgewählt wird.

4. Verfahren nach einem der Ansprüche 1 oder 2, dadurch gekennzeichnet, daß man die Teilchen des Gemisches bei einer Temperatur von 60 bis 90 °C in Bewegung versetzt.

5. Verfahren nach einem der Ansprüche 1 oder 2, dadurch gekennzeichnet, daß man ein sofort lösliches Magermilchpulver mit einem Wassergehalt von 2 bis 4 % einsetzt.

6. Verfahren nach Anspruch 2, dadurch gekennzeichnet, daß man 5 bis 10 % Kakao mit dem Magermilchpulver vermischt.

7. Verfahren nach einem der Ansprüche 2 oder 3, dadurch gekennzeichnet, daß man 0 4 bis 10 Gewichts-% des pharmazeutischen Wirkstoffes mit dem Magermilchpulver vermischt und anschließend so viel Magermilchpulver zusetzt, daß man eine Wirkstoffkonzentration von 0,1 bis 1 Gewichts-% erreicht.

8. Verfahren nach einem der Ansprüche 1 oder 2, dadurch gekennzeichnet, daß man die Teilchen des Gemisches mit einer elektrischen Flügelmühle 15 bis 120 Sekunden lang in Bewegung versetzt.

9. Verfahren nach einem der Ansprüche 1 oder 2, dadurch gekennzeichnet, daß man die Teilchen des Gemisches mit Hilfe eines Mittels für ein mäßiges Rühren während einer Zeit von 15 bis 120 Minuten in Bewegung versetzt.

FIG.1

EP 0 196 434 B1

FIG. 2

EP 0 196 434 B1